(19) Europäisches Patentamt
European Patent Office
Office européen des brevets

(11) EP 1 157 693 A1

(12) EUROPEAN PATENT APPLICATION

(43) Date of publication:
**28.11.2001 Bulletin 2001/48**

(21) Application number: **00110160.9**

(22) Date of filing: **12.05.2000**

(51) Int Cl.[7]: **A61K 31/353**, A61P 29/00, A61P 37/08, A61P 11/06, A61P 27/14

(84) Designated Contracting States:
**AT BE CH CY DE DK ES FI FR GB GR IE IT LI LU MC NL PT SE**
Designated Extension States:
**AL LT LV MK RO SI**

(71) Applicants:
- **National Agricultural Research Organisation (NARO)**
  **Ibaraki 305-8517 (JP)**
- **Bio-oriented Technology Research Advancement Institution**
  **Omiya-shi, Saitama 331-8537 (JP)**

(72) Inventors:
- **Yamamoto, Mari**
  **Kakegawa-shi, Shizuoka 436-0911 (JP)**
- **Sano, Mitsuaki**
  **Shizuoka-shi, Shizuoka 420-0911 (JP)**
- **Miyase, Toshio**
  **Shizuoka-shi, Shizuoka 422-8041 (JP)**
- **Kawahara, Hiroharu**
  **Kokurakita-ku, Kitakyushu-shi, Fukuoka (JP)**
- **Tsuji, Kenkou**
  **Haibara-gun, Shizuoka 428-0039 (JP)**

(74) Representative:
**Gille Hrabal Struck Neidlein Prop Roos**
**Patentanwälte**
**Brucknerstrasse 20**
**40593 Düsseldorf (DE)**

(54) **USE OF EPIGALLOCATECHIN 3-O-(3-O-METHYL) GALLATE AND/OR EPIGALLOCATECHIN 3-O-(4-O-METHYL) GALLATE FOR THE TREATMENT OF ALLERGY AND/OR INFLAMMATION**

(57) Therapeutic and preventive drug is provided, which have not any side effect, which is highly safe upon repeated uses for a long period of time, and which are useful in daily food and drink and cosmetics. Antiallergic agents, anti-inflammatory agent, food and drink and cosmetics, which are characterized in that epigallocatechin 3-O-(3-O-methyl) gallate and(or) epigallocatechin 3-O-(4-O-methyl) gallate are/is contained as active ingredients.

EP 1 157 693 A1

## Description

### FIELD OF THE INVENTION

The present invention relates to a drug containing as active ingredients catechin derivatives such as epigallocatechin 3-O-(3-O-methyl) gallate and epigallocatechin 3-O-(4-O-methyl) gallate, and also relates to food and drink and a cosmetic containing the same. More specifically, the present invention is directed to a drug, food and drink, and to a cosmetic having activities to suppress immediate type and delayed type allergies and to alleviate inflammation.

### BACKGROUND OF THE INVENTION

In recent years, there has been observed an increase in allergic diseases, and it was reported that one third of born children suffer from atopic dermatitis or asthma. In addition, a drastic increase in crisis of pollinosis has become a big social problem.

It is considered that causes of such an increase in allergic conditions reside in changes in environments surrounding us such as westernized eating habits, uses of food additives, air pollution and excess stress.

Allergic reactions are classified into types I to IV based on its relevant immunological competent cell and immunoglobulin. Diseases represented by allergic rhinitis and bronchial asthma belong to type I allergic reaction, wherein chemical mediators such as histamine, leukotrienes and prostaglandins are produced and released from mast cells or basophils via IgE cross-linking, hyperfunction in blood vessel permeability, contraction of bronchial smooth muscles, stimulus in nerve terminals, etc. Therefore, for treatment of type I allergic diseases, there are used antihistaminic agents and antiallergic agents having an activity to suppress release of chemical mediators from mast cells.

Antihistaminic agents and basic antiallergic agents have, however, side effects such as sleepiness, thirst and gastrointestinal disorders, and thus the repeated use thereof for a long period of time becomes a problem.

An type IV allergic reaction is a delayed type one to which T cells are related, wherein T cells received an antigen information via antigen presenting cells such as Langerhans' cells and macrophages, produce and release various cytokines to cause a delayed type inflammatory reaction due to accumulation of eosinophils and macrophages.

Allergic contact dermatitis is a typical disease causedbased on the type IV allergic reaction. For the treatment of the type IV allergic diseases, steroid agents are used, which suppress cytokine production in T cells and show dramatic effects on the treatment of eczema. On the contrary, they can cause heavy side effects such as deterioration in adrenal cortical function, skin flushing, atrophy and angiotelectasis.

On the other hand, tea is a typical luxurious beverage, which has been drunk for over 2,000 years by huge number of people. In addition, it has been found that tea has various physiological functions, and antioxidative, antitumor, carcinogenic suppressive, antibacterial, antiviral activities and preventative action for dental caries have been reported, for instance.

As to allergic activities, examples of treating drugs for type I allergic reaction containing as a main ingredient an extract of oolong tea with using an activity to suppress histamine release from mast cells as an indicator are described in Japanese Patent Application Laid-open No. Hei 3-258726; examples of natural caffeine effective for rising reaction of blood vessel permeability in type I allergic conditions are described in Japanese Patent Application Laid-open No. Hei 7-17865; and examples of antiallergic agents, anti-inflammatory agents, anti-atopic dermatitic agents and antipsoriatic agents containing as active ingredients extracts from oolong tea are described in Japanese Patent Application Laid-open Nos. Hei 10-77231 and Hei 10-175874.

Further, it has been reported that green tea catechins such as epigallocatechin gallate and epicatechin gallate suppress histamine release from mast cells in abdominal cavity of rats (Journal of Japanese Society for Food Science and Technology, Vol. 42, No. 11, pp 952-958, 1995; and Allergy, Vol. 52, No.1, pp 58-64, 1997). There has not been, however, reported that catechin derivatives such as epigallocatechin 3-O-(3-O-methyl) gallate and epigallocatechin 3-O-(4-O-methyl) gallate suppress type I and type IV allergic reactions. Further, there is not exemplified isolation of epigallocatechin 3-O-(4-O-methyl) gallate from tea leaves.

Although drugs in compliance with allergic conditions have been developed and used for the treatment, these have side effects. Thus, there has been strongly desired to develop antiallergic agents derived from natural origins with high safety and without any side effect even after used for a long period of time.

### SUMMARY OF THE INVENTION

Therefore, an object of the present invention is to provide a therapeutic and preventive drug for allergic diseases which have not any side effect, which is highly safe even after used for a long period of time, and can be used in daily used products such as food and drink and cosmetics.

The inventors of the present invention have found that catechin derivatives such as epigallocatechin 3-O-(3-O-methyl) gallate and epigallocatechin 3-O-(4-O-methyl) gallate have the suppressive effects described below by screening substances having antiallergic activity with using, as indications, a suppressive effect on histamine release that is an experimental model for type I allergic reaction (in vitro method), an in vivo mouse abdominal wall method (AW method) and a suppressive effect on oxazolone-derived mouse auricula dermatitis that is an experimental model for type IV allergic reaction in order to solve the above-mentioned problem, and they have attained the present invention based on this finding.

The present invention according to the first aspect of the invention relates to an antiallergic agent, characterized in that the antiallergic agent contains as active ingredients epigallocatechin 3-O-(3-O-methyl) gallate and/or epigallocatechin 3-O-(4-O-methyl) gallate.

The present invention according to the second aspect of the invention relates to an anti-inflammatory agent, characterized in that the anti-inflammatory agent contains as active ingredients epigallocatechin 3-O-(3-O-methyl) gallate and/or epigallocatechin 3-O-(4-O-methyl) gallate.

The present invention according to the third aspect of the invention relates to an antiallergic agent as set forth in the first aspect of the invention, characterized in that the antiallergic agent is an oral antiallergic agent.

The present invention according to the fourth aspect of the invention relates to an anti-inflammatory agent as set forth in the second aspect of the invention, characterized in that the anti-inflammatory agent is an oral anti-inflammatory agent.

The present invention according to the fifth aspect of the invention relates to a food including an oral antiallergic agent or an oral anti-inflammatory agent characterized by containing as active ingredients epigallocatechin 3-O-(3-O-methyl) gallate and/or epigallocatechin 3-O-(4-O-methyl) gallate.

The present invention according to the sixth aspect of the invention relates to a cosmetic including an external antiallergic agent or an external anti-inflammatory agent characterized by containing as active ingredients epigallocatechin 3-O-(3-O-methyl) gallate and/or epigallocatechin 3-O-(4-O-methyl) gallate.

The present invention according to the seventh aspect of the invention relates to a method for preventing, suppressing or alleviating an allergic condition or an inflammatory condition, characterized by dosing an antiallergic agent as set forth in the first aspect of the invention or an anti-inflammatory agent as set forth in the second aspect of the invention to a patient who requires prevention, suppression or alleviation of the allergic condition or the inflammatory condition.

## BRIEF DESCRIPTION OF THE DRAWINGS

In the accompanying drawings:

Fig. 1 shows an extracting operation of polyphenol fraction from tea leaves;
Fig. 2 shows an isolating operation of epigallocatechin 3-O-(3-O-methyl) gallate or epigallocatechin 3-O-(4-O-methyl) gallate from tea leaves;
Fig. 3 is a graph showing effects of various catechin derivatives on suppression of histamine release from mouse mast cells.

## DETAILED DESCRIPTION OF THE INVENTION

epigallocatechin 3-O-(3-O-methyl) gallate and epigallocatechin 3-O-(4-O-methyl) gallate used in the invention are those expressed by the following structural formulae, but other catechin derivatives such as epigallocatechin 3-O-(5-O-methyl) gallate or mixtures thereof may be used in the invention.

Tea (camellia sinensis) has been daily drunk from ancient for a long period, and it has been recognized as a very safe beverage having no adverse effect on human body. Therefore, catechin derivatives derived from tea leaves such as epigallocatechin 3-O-(3-O-methyl) gallate and epigallocatechin 3-O-(4-O-methyl) gallate to be used in the present invention are acceptable with a sense of security.

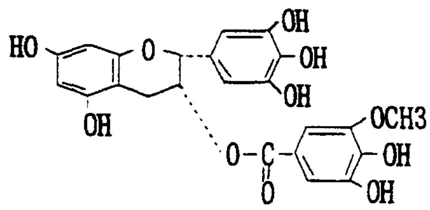

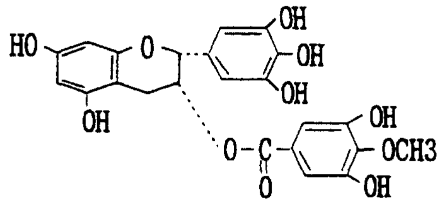

Catechin derivatives derived from tea leaves such as epigallocatechin 3-O-(3-O-methyl) gallate and epigallocatechin 3-O-(4-O-methyl) gallate to be used in the present invention can be separated and collected from a polyphenol fraction, which is in turn obtained by extracting tea leaves such as "Seishin-Taipan", "Benihomare", "Benifuji" and "Benifuki" tea leaves, preferably dried tea leaves, with an aqueous solvent. Due consideration of the fact that the thus obtained extract is used finally as a component in food and drink or cosmetics etc., it is preferable to use water, ethanol or mixtures thereof as the solvent from the viewpoint of safety.

Raw materials, tea leaves, for extraction are preferably ones dried by means of microwave, wherein a ratio (by weight) of tea leaves to the solvent is preferably from 5 to 100 parts of the solvent to 1 part of tea leaves. The extraction temperature is not specifically limited, and in general it is convenient for operation to be within a range from the room temperature to the boiling point of the solvent under the normal pressure. The extraction period is preferably within a range of from 10 minutes to 6 hours.

Catechin derivatives such as epigallocatechin 3-O-(3-O-methyl) gallate and epigallocatechin 3-O-(4-O-methyl) gallate to be used in the present invention can be dosed orally as such in the form of extracts or after diluted with water etc. appropriately, as antiallergic agents or anti-inflammatory agents. Further, they may be prepared by manufacturing them with conventionally used pharmaceutical carriers. For example, the above-mentioned extracts may be formulated into oral liquors such as syrup or into oral solid drugs such as tablets, capsules, granules and powders by processing them into extracts or powders and blending them with pharmaceutically acceptable carriers. Herein, as pharmaceutically acceptable carriers, there may be used various organic and inorganic carrier substances conventionally used as manufacturing bases. They may be blended as vehicles, lubricants, binders and disintegrators for solid drugs, or as solvents, vehicles, suspending agents and binders for liquid drugs. Additionally, there may be used additives optionally such as antioxidants, antiseptics, colorants and sweetening agents.

As the above-mentioned manufacturing bases, there may be used conventional ones. As vehicles, there may be mentioned for example sucrose, lactose, D-mannitol, starch, crystalline cellulose and light anhydrous silicic acid etc. As lubricants, there may be mentioned for example magnesium stearate, calcium stearate, talc and colloidal silica etc. Further, as binders, there may be mentioned for example cellulose, sucrose, D-mannitol, dextrin and polyvinylpyrrolidone etc.

Next, as disintegrators, there may be mentioned for example polyethylene glycol, D-mannitol, benzyl benzoate, ethanol, sodium carbonate and sodium citrate etc. As solvents, there may be mentioned for example purified water, ethanol and propylene glycol etc. As suspending agents, there may be mentioned for example surfactants such as sodium lauryl sulfate, lecithin and glycerol monostearate as well as hydrophilic polymers such as polyvinyl alcohol, carboxymethyl cellulose and hydroxymethyl cellulose.

Further, as antioxidants, there may be mentioned for example sulfites and ascorbic acid etc. As antiseptics, there may be mentioned for example para-oxybenzoates, benzyl alcohol and sorbic acid etc. As colorants, there may be mentioned various food colorings.

Next, food and drink according to the present invention have not been limited. For making various kinds of food and drink, the tea leaf extracts containing the above-mentioned catechin derivatives may be used as such or as extracts and powders, or they may be also used by combination with food or drink materials or carriers generally

acceptable for preparation of food and drink. For example, as beverages, there may be mentioned carbonic beverages, fruit beverages and lactic acid bacteria beverages. Further, as confectionery, there may be mentioned biscuits, chocolates, candies, chewing gums and snacks. As foods, there may be mentioned bread, noodles, soybean processed products, dairy products, meat products, boiled fish pastes, dressings, mayonnaise, curry and soup.

As carriers acceptable for preparation of food and drink, there may be mentioned conventional ones, for example, sweetening agents such as sucrose, glucose, isomerized liquid sugars and oligosaccharides, as well as seasonings, sour agents, various colorants, preservatives, thickening agents, antioxidants, cure accelerator, bleaching agents, quality modifiers, inflating agents and emulsifiers etc., which may be used appropriately.

In the case that catechin derivatives or tea leaf extracts containing them are formulated into cosmetics, they may be converted into solutions, extracts, suspensions, emulsions, aerosols, ointments, cataplasms and lotions by diluting for example with water, concentrating, or pulverizing or granulating and then formulating with known pharmaceutical bases or carriers. Herein, as known pharmaceutical bases or carriers, there may be mentioned aqueous components, surfactants, oily components, solubilizing agents, humectants, powdery components, alcohols, antiseptics, antioxidants, thickeners, pigments and perfumes etc., which may be appropriately selected if required for manufacturing the objective forms.

As external drugs for skin, they may be for example took into forms of lotions, gels, emulsions and ointments etc., from which the following various forms of cosmetics may be prepared; make-up cosmetics such as skin lotions, creams, milky lotions, face cleansings, skin cleansings and lipsticks; hair cosmetics such as shampoo, hair conditioners and hair tonics; and bathing agents such as bath oils.

In the present invention, catechin derivatives such as epigallocatechin 3-O-(3-O-methyl) gallate and epigallocatechin 3-O-(4-O-methyl) gallate may be used in appropriate amounts due consideration of the objects of use. For example, in the case of antiallergic agents and anti-inflammatory agents, the amount of the catechin derivative is suitably 5-100 mg/kg, preferably 10-50 mg/kg, per a day. A polyphenol fraction containing the catechin derivative is suitably used in an amount of 10-300 mg/kg, preferably 20-100 mg/kg, per a day.

Further, in the case of using as food and drink, the polyphenol fraction containing the above-mentioned catechin derivative as an effective ingredient is used in an amount of 5-300 mg/kg, preferably 20-100 mg/kg, per a day. Further, in the case of cosmetics, the polyphenol fraction containing the above-mentioned catechin derivative as an effective ingredient is used in an amount of 5-300 mg/kg, preferably 20-100 mg/kg, per a day, similarly.

In all cases, they may be used once a day or may be used by dividing them into several times a day.

Antiallergic agents and anti-inflammatory agents according to the present invention are effective for prevention, suppression or alleviation of conditions based on inflammation or allergic reaction. In particular, they are effective for prevention, suppression or alleviation of conditions such as atopic dermatitis.

The agents according to the present invention have catechin derivatives derived from tea leaves such as epigallocatechin 3-O-(3-O-methyl) gallate and epigallocatechin 3-O-(4-O-methyl) gallate as active ingredients, which are superior in safety and which have not any side effect in the case of repeated uses for a long period. Further, they are useful for prevention or alleviation of conditions due to allergic reaction by daily taking food and drink containing tea leaf extracts, in which main components are these active ingredients. Further, the antiallergic agents and anti-inflammatory agents may be applied as external agents for skin and they have alleviation and suppressive effects on inflammation due to allergic reaction. Therefore, they can be used daily by including them in cosmetics.

## EXAMPLES

Typical examples and experimental examples are shown as follows in order to illustrate the invention in more detail, but the invention is not limited only thereto.

### Experimental Example 1

Extraction of polyphenol fractions (tea extracts) from various tea leaves

As shown in Fig. 1, 50 g of tea leaves dried by means of microwave were extracted in 1000 ml of boiling distilled water for 1 hour and thereafter the solubles were filtered with filter papers. The filtrate was passed through a column (5x20 cm) filled with Mitsubishi Diaion HP-20, a porous polystyrene resin. After washing the column with purified water, a fraction eluted in 50% methanol was furthermore passed through an Amberlyst 15 (4x20 cm), to obtain a polyphenol fraction without caffeine that is eluted in methanol.

Experimental Example 2

Isolation of epigallocatechin 3-O-(3-O-methyl) gallate and epigallocatechin 3-O-(4-O-methyl) gallate

Polyphenol fractions of "Seishin-Taipan" tea leaves dried by means of microwave were fractionated by the method shown in Fig. 2, to isolate the above-mentioned components. That is, 300 g of tea leaves were subjected to fractionation of polyphenol, to obtain 31 g of pale brown powders.

The polyphenol fraction was passed through a column (9 cm x 25 cm) filled with silica gel and eluted with chloroform-methanol-water type solvents. A fraction A (216 mg) eluted with chloroform-methanol-water (82:16:2); fractions B (1708 mg), C (730 mg), D (1927 mg), E (7020 mg) and F (914 mg) eluted with the same component solvent (80:18:2); and a fraction G (13250 mg) eluted with the same component solvent (40:50:10) were obtained.

Then, the fractions C and D were furthermore fractionated by liquid chromatography with use of reversed phase columns (5 cm x 50 cm, ODS, two columns being connected), to obtain epigallocatechin 3-O-(3-O-methyl) gallate (241 mg) and epigallocatechin 3-O-(4-O-methyl) gallate (101 mg) from the fraction eluted in acetonitrile-water (12:88) as colorless powders, respectively.

Experimental Example 3

Determination of antiallergic activity

As an evaluation means for antiallergic activity in vitro, there was used a method to determine an amount of histamine release during degranulation of mast cells that is a standard testing method for type I allergy. Further, as an evaluation means for antiallergic activity in vivo, there were used an abdominal wall method that evaluates anapylaxy reaction in mouse abdominal wall with use of ovoalbumin (OVA) as a sensitizing drug for type I and an oxazolone-derived auricula edema method for type IV.

1) Immediate type allergy (Type I)

1-1) Histamine release test (in vitro test)

As a judging method for type I allergy, there was used an activity to suppress histamine release from mouse mast cells as an indicator. That is, MC/9 (ATCC CRL1649) was used as mouse mast cells, which were cultured in RPMI 1640 medium supplemented with 10% of heat-inactivated FBS, 10% of WEHI-3b culture supernant, 2 mM of glutamine and 50 μM of 2-mercaptoethanol in humidified 95% air/5% $CO_2$ at 37°C.

After the cells ($1x10^7$ cells/ml) were sensitized with anti-DNP-mouse IgE antibody for one night, they were incubated together with a test sample with floating on a Tyrode solution. Thereafter, DNP-BSA (antigen) was added to them to induce degranulation, and an amount of histamine in the supernant was determined by on-column high performance liquid chromatography method.

The antiallergic activity was judged as high if the relative value against the control, phosphate buffered saline (PBS) is low.

1-2) Mouse abdominal wall method (in vivo test)

A method for investigating a hyperfunction in blood vessel permeability in mouse abdominal wall that is a judging method for immediate type allergy (Type I) (Biol. Pharm. Bull., vol. 20, pp 714-716, 1997) was modified partly and used as an evaluation method for antiallergic activity. That is, sensitization was carried out by dosing 50 μl of a mixed solution (1:1) of OVA (2 mg/ml) and incomplete Freund adjuvant in abdominal cavity of mouse (ddY, male, 5 weeks). Allergy was induced by dosing 0.1 ml of 2% Evans blue solution intravenously, without anesthesia, to the mouse 9 days after sensitization, immediately thereafter pealing off skins quickly under ether anesthesia and injecting 50 μl of an inducing sample solution (OVA 100 μg/ml) in abdominal wall. 7 minutes after induction, the mouse was subjected to vertebral dislocation and ablation of abdominal wall. A portion of leaked pigment was marked and an area thereof was read by a densitography, from which a leaked area was measured by means of computer image analysis. The test sample or the phosphate buffered saline (control group) was dosed orally sixty minutes before induction by means of gastric probe. Evaluation of activity is expressed as a suppression percentage of pigment leaked area to that of the control group.

2) Delayed type allergy (Type IV)

It was carried out according to a delayed type allergic dermatitis testing method by means of oxazolone. That is, hair in an abdominal part of a mouse (ICR, male, 5 weeks) was cut and 100 μl of ethanol solution of 0.5% oxazolone was coated on the cut part to carry out sensitization. 5 days after sensitization, a test sample was formulated in an acetone solution of 0.5%. oxazolone, 10 μl of which was coated on a right side auricula to induce allergy (the coated test sample was 0.128 mg/auricula).

24 hours after induction, the mouse was killed with ether anesthesia. Concave parts were perforated in right and left auriculae with a punch having a hole diameter of 5 mm. The parts were weighed, and an swelling percentage of auriculae was calculated according to the following equation.

[Equation 1]

$$\text{Swelling percentage of auricula} = 100 \times (R-L)/L$$

wherein, L is weight of left side normal auricula, and R is weight of right side edema auricula.

Further, apart from the weight method, a thickness of the right auricula was determined additionally before and after edema induction by means of slide calipers under ether anesthesia, to determine an swelling percentage according to the following equation. Hydrocortisone was used as a positive control.

[Equation 2]

$$\text{Swelling percentage of auricula} = 100 \times (A-B)/B$$

wherein, A is thickness of auricula after edema induction, and B is thickness of auricula before edema induction.

Experimental Example 4

Properties of epigallocatechin 3-O-(3-O-methyl) gallate and epigallocatechin 3-O-(4-O-methyl) gallate

Properties of both compounds are shown as follows.

1) epigallocatechin 3-O-(3-O-methyl) gallate

colorless amorphous powder
molecular formula; $C_{23}H_{20}O_{11}$
molecular weight; 472

$^{1}$H-NMR (400 MHz, acetone-$d_6$) d: 2.96 (1H, dd, J=17.5 and 3 Hz, H-4), 3.04 (1H, dd, J=17.5 and 4 Hz, H-4), 3.82 (3H, s, $OCH_3$), 5.10 (1H, br s, H-2), 5.52 (1H, m, H-3), 6.03 (1H, d, J=2 Hz, H-8), 6.04 (1H, d, J=2 Hz, H-6), 6.64 (2H, s, H-2' and H-6'), 7.06 (1H, d, J=2 Hz, H-2"), 7.12 (1H, d, J=2 Hz, H-6")

$^{13}$C-NMR (100 MHz, acetone-$d_6$) d: 26.3 (C-4), 56.5 ($OCH_3$), 69.9 (C-3), 78.0 (C-2), 95.7 (C-8), 96.4 (C-6), 98.9 (C-4a), 106.0 (C-2"), 106.6 (C-2' and C-6'), 111.6 (C-6"), 121.7 (C-1"), 130.8 (C-1'), 133.1 (C-3' and C-5'), 145.7 (C-4'), 146.3 (C-4"), 148.5 (C-5"), 149.6 (C-3"), 157.0 (C-8a), 157.4 (C-5), 157.8 (C-7), 166.3 (C=O)

2) epigallocatechin 3-O-(4-O-methyl) gallate

colorless amorphous powder

molecular formula; $C_{23}H_{20}O_{11}$
molecular weight; 472

$^1$H-NMR (400 MHz, acetone-$d_6$) d: 2.93 (1H, dd, J=17.5 and 2.5 Hz, H-4), 3.04 (1H, dd, J=17.5 and 4.5 Hz, H-4), 3.83 (3H, s, $OCH_3$), 5.06 (1H, br s, H-2), 5.54 (1H, m, H-3), 6.03 (1H, d, J=2 Hz, H-8), 6.05 (1H, d, J=2 Hz, H-6), 6.64 (2H, s, H-2' and H-6'), 7.00 (2H, s, H-2" and H-6")
$^{13}$C-NMR (100 MHz, acetone-$d_6$) d: 26.5 (C-4), 60.5 ($OCH_3$), 69.8 (C-3), 78.0 (C-2), 95.7 (C-8), 96.4 (C-6), 98.8 (C-4a), 106.6 (C-2' and C-6'), 109.9 (C-2" and C-6"), 126.4 (C-1"), 130.7 (C-1'), 133.0 (C-3' and C-5'), 140.5 (C-4"), 146.2 (C-4'), 151.0 (C-3" and C-5"), 157.0 (C-8a), 157.4 (C-5), 157.8 (C-7), 166.0 (C=O)

$[a]^{23}_D$: $-156.6_o$ (c=0.90, MeOH)
FAB-MS: 473$[M+H]^+$

Experimental Example 5

Suppressive effect on type I allergy (in vitro test)

Results of antiallergic activity on histamine release from mouse mast cells by administering epigallocatechin gallate (abbreviated as EGCG), epigallocatechin 3-O-(3-O-methyl) gallate (abbreviated as EGCG3"Me) or epigallocatechin 3-O-(4-O-methyl) gallate (abbreviated as EGCG4"Me) are shown in Fig. 3.
As apparent from the figure, EGCG4"Me expressed a very strong suppressive activity on histamine release.

Experimental Example 6

Suppressive effect on Type I allergy (in vivo test)

Results of antiallergic activity on OVA sensitized by administering EGCG, EGCG3"Me or EGCG4"Me are shown in Table 1.
As clear from Table 1, EGCG3"Me and EGCG4"Me showed higher activity than EGCG at 10 mg/kg oral dosage in every case.

[Table 1]

Table 1:

| Antiallergic activity (Type I) of EGCG3"Me and EGCG4"Me | | | |
|---|---|---|---|
| Test sample | Dosage (mg/kg) | Intravenous dose suppressive percentage (%) | Oral dose suppressive percentage (%) |
| Diphenhydramine* | 1 | 59.0 ± 14.3 | 75.3 ± 15.4 |
| EGCG | 10 | - | 12.6 ± 4.4 |
| | 50 | 21.7 ± 14.9 | 26.9 ± 4.6 |
| | 100 | 25.4 ± 11.1 | 22.1 ± 6.7 |
| EGCG3"Me | 10 | 22.1 ± 5.2 | 36.4 ± 2.1 |
| | 50 | 40.9 ± 5.2 | 60.8 ± 5.0 |
| EGCG4"Me | 10 | 23.1 ± 5.2 | 50.1 ± 5.4 |
| | 50 | 46.6 ± 6.2 | 58.8 ± 6.4 |

*: Positive control, -: No effect, Average ± Standard error

Further, as shown in Table 2, EGCG4"Me showed high activity at 10 mg/kg oral dosage.

[Table 2]

Table 2:

| Comparison of effects by oral dose | | | | |
|---|---|---|---|---|
| dosage | | | | |
| | 10 mg/kg | | 50 mg/kg | |
| | EGCG3"Me | EGCG4"Me | EGCG3"Me | EGCG4"Me |
| EGCG | P<0.01 | P<0.01 | P<0.01 | P<0.01 |
| EGCG3"Me | - | P<0.01 | - | NS |

NS: No significant difference

Experimental Example 7

Effect on delayed type allergy (Type IV)

Results of antiallergic activity on oxazolone-sensitized mouse by dosing epicatechin (abbreviated as EC), EGCG, EGCG3"Me or EGCG4"Me are shown in Table 3.

As clear from the Table, EGCG4"Me showed a relatively higher suppressive effect than EGCG and EGCG3"Me for evaluation of auricula thickness but there was not recognized such tendency for evaluation of auricula weight.

[Table 3]

Table 3:

| Effects on delayed type allergy (Type IV) | | | | |
|---|---|---|---|---|
| Group | Evaluation by auricula thickness | suppressive percentage (%) | Evaluation by auricula weight | suppressive percentage (%) |
| Control (non-dosed group) | 70.6 ± 3.0 | - | 72.3 ± 2.9 | - |
| Hydrocortisone | 5.7 ± 1.9** | 92 | 11.7 ± 2.8** | 84 |
| EC | 20.6 ± 7.2* | 71 | 34.1 ± 11.7* | 53 |
| EGCG | 18.1 ± 5.5** | 74 | 7.7 ± 0.8** | 89 |
| EGCG3"Me | 16.8 ± 6.9** | 76 | 19.4 ± 3.4** | 73 |
| EGCG4"Me | 8.6 ± 1.9** | 88 | 16.2 ± 5.0** | 77 |

Average ± Standard error *:P<0.05
**:P<0.01(by Mann-Whitney's U-test)

Example 1

Preparation of a candy

The following materials were used to prepare a candy according to the conventional method:

| | |
|---|---|
| powdery sorbitol | 99.25 g |
| perfume | 0.2 g |
| sorbitol seed | 0.05 g |
| polyphenol fraction* | 0.5 g |
| total | 100 g |

*: containing EGCG3"Me and EGCG4"Me.

Example 2

Preparation of a chewing gum

The following materials were used to prepare a chewing gum according to the conventional method:

| | |
|---|---|
| gum base | 20 g |
| sodium carbonate | 2 g |
| stevioside | 0.1 g |
| polyphenol fraction* | 0.5 g |
| lactose | 76.4 g |
| perfume | 1 g |
| total | 100 g |

*: containing EGCG3"Me and EGCG4"Me.

Example 3

Preparation of a bathing agent containing a tea leaf extract having a catechin derivative such as EGCG3"Me and EGCG4"Me as a main component

| | |
|---|---|
| dried sodium sulfate | 53 g |
| sodium hydrogen carbonate | 40 g |
| light calcium carbonate | 1 g |
| anhydrous silicic acid | 1 g |
| polyphenol fraction* | 5 g |
| perfume | trace |
| pigment | trace |
| total | 100 g |

*: containing EGCG3"Me and EGCG4"Me.

## Claims

1. An antiallergic agent, wherein the antiallergic agent contains as active ingredients epigallocatechin 3-O-(3-O-methyl) gallate and/or epigallocatechin 3-O-(4-O-methyl) gallate.

2. An anti-inflammatory agent, wherein the anti-inflammatory agent contains as active ingredients epigallocatechin 3-O-(3-O-methyl) gallate and/or epigallocatechin 3-O-(4-O-methyl) gallate.

3. An antiallergic agent according to claim 1, wherein the antiallergic agent is an oral antiallergic agent.

4. An anti-inflammatory agent according to claim 2, wherein the anti-inflammatory agent is an oral anti-inflammatory agent.

5. A food comprising an oral antiallergic agent or an oral anti-inflammatory agent containing as active ingredients epigallocatechin 3-O-(3-O-methyl) gallate and/or epigallocatechin 3-O-(4-O-methyl) gallate.

6. A cosmetic comprising an external antiallergic agent or an external anti-inflammatory agent containing as active ingredients epigallocatechin 3-O-(3-O-methyl) gallate and/or epigallocatechin 3-O-(4-O-methyl) gallate.

7. A method for preventing, suppressing or alleviating an allergic condition or an inflammatory condition, which comprises dosing an antiallergic agent according to claim 1 or an anti-inflammatory agent according to claim 2 to a patient who requires prevention, suppression or alleviation of the allergic condition or inflammatory condition.

FIG. 1

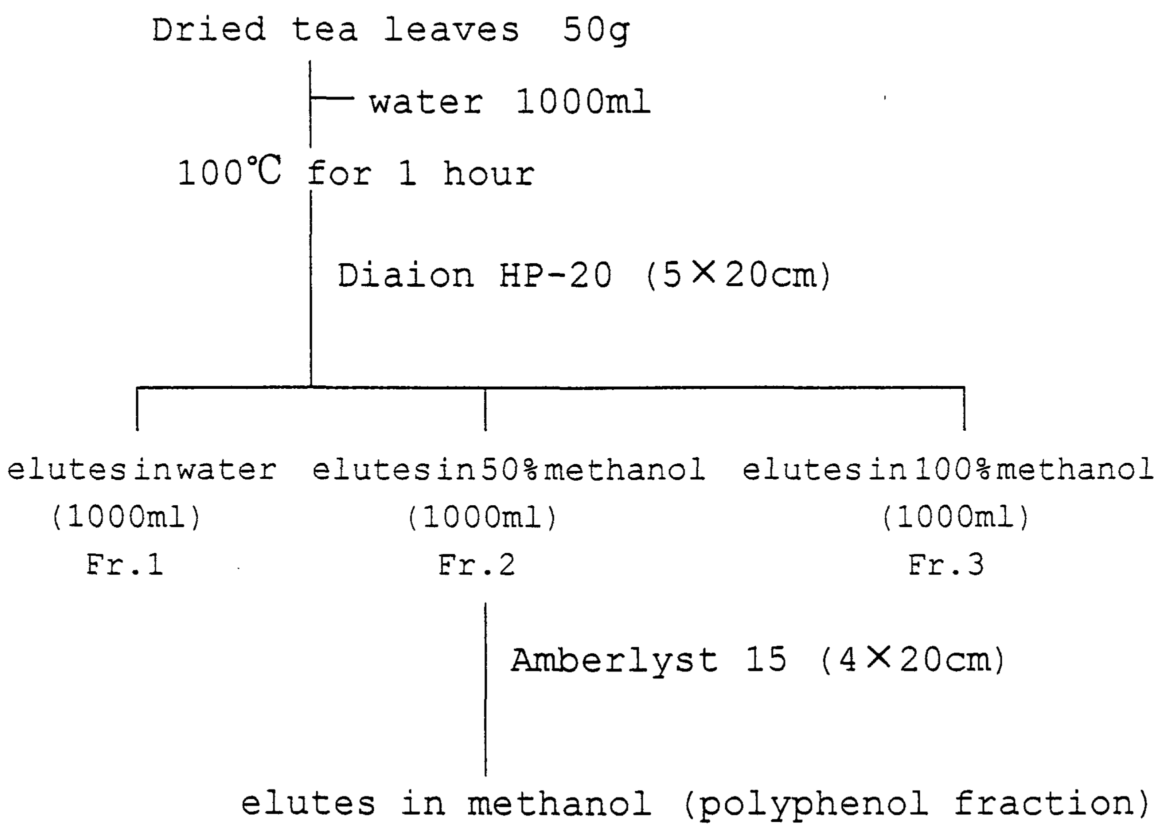
Dried tea leaves 50g
water 1000ml
100℃ for 1 hour
Diaion HP-20 (5×20cm)
elutes in water
(1000ml)
Fr.1
elutes in 50% methanol
(1000ml)
Fr.2
elutes in 100% methanol
(1000ml)
Fr.3
Amberlyst 15 (4×20cm)
elutes in methanol (polyphenol fraction)

*FIG. 2*

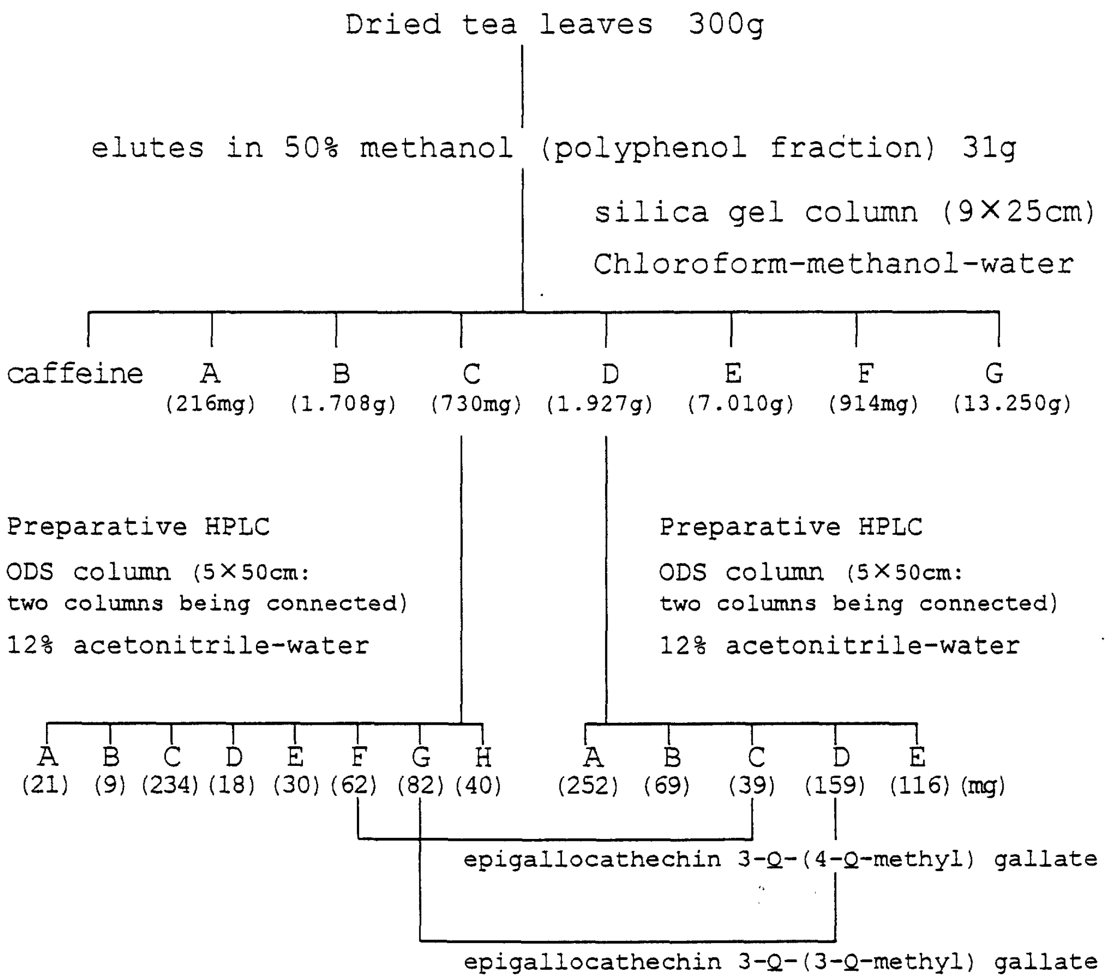
Dried tea leaves 300g
elutes in 50% methanol (polyphenol fraction) 31g
silica gel column (9×25cm)
Chloroform-methanol-water
caffeine
A (216mg)
B (1.708g)
C (730mg)
D (1.927g)
E (7.010g)
F (914mg)
G (13.250g)
Preparative HPLC
ODS column (5×50cm: two columns being connected)
12% acetonitrile-water
Preparative HPLC
ODS column (5×50cm: two columns being connected)
12% acetonitrile-water
A (21)
B (9)
C (234)
D (18)
E (30)
F (62)
G (82)
H (40)
A (252)
B (69)
C (39)
D (159)
E (116) (mg)
epigallocathechin 3-O-(4-O-methyl) gallate
epigallocathechin 3-O-(3-O-methyl) gallate

FIG. 3

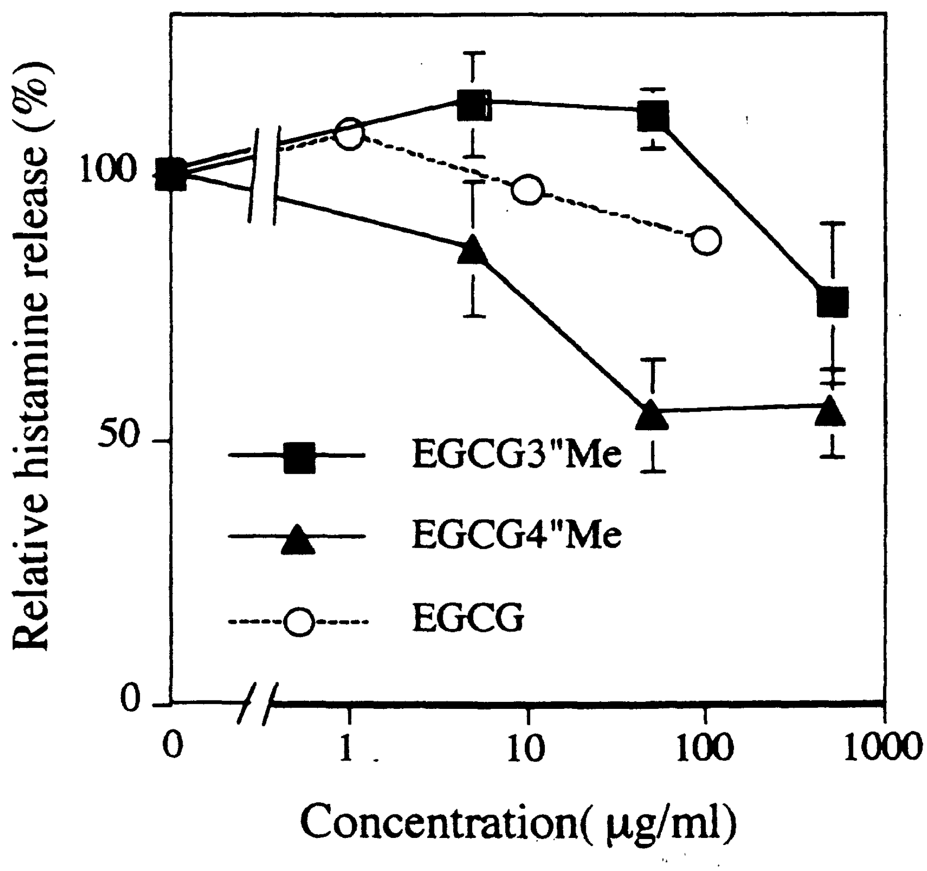
Relative histamine release (%)
100
50
0
EGCG3"Me
EGCG4"Me
EGCG
0
1
10
100
1000
Concentration( μg/ml)

European Patent Office

**PARTIAL EUROPEAN SEARCH REPORT**

which under Rule 45 of the European Patent Convention shall be considered, for the purposes of subsequent proceedings, as the European search report

Application Number
EP 00 11 0160

**DOCUMENTS CONSIDERED TO BE RELEVANT**

| Category | Citation of document with indication, where appropriate, of relevant passages | Relevant to claim | CLASSIFICATION OF THE APPLICATION (Int.Cl.7) |
|---|---|---|---|
| X | DATABASE CHEMABS 'Online!<br>CHEMICAL ABSTRACTS SERVICE, COLUMBUS, OHIO, US;<br>AN: 132:262597,<br>XP002149538<br>* abstract *<br>& YAMAMOTO-MAEDA, M. ET AL: "Novel ant-allergic factors in tea leaves"<br>BAIOSAIENSU TO INDASUTORI,<br>vol. 57, no. 10, 1999, pages 691-692,<br>--- | 1-7 | A61K31/353<br>A61P29/00<br>A61P37/08<br>A61P11/06<br>A61P27/14 |
| X | TACHIBANA, H. ET AL: "Identification of a methylated tea catechin as an inhibitor of degranulation in human basophilic KU812 cells"<br>BIOSCI. BIOTECHNOL. BIOCHEM.,<br>vol. 64, no. 2, 2000, pages 452-454,<br>XP000946324<br>* the whole document *<br>---<br>-/-- | 1-7 | |
| | | | **TECHNICAL FIELDS SEARCHED (Int.Cl.7)** |
| | | | A61K |

**INCOMPLETE SEARCH**

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 7 is directed to a method of treatment of the human/animal body (Article 52(4) EPC), the search has been carried out and based on the alleged effects of the compounds.

| Place of search | Date of completion of the search | Examiner |
|---|---|---|
| THE HAGUE | 6 October 2000 | Mair, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 00 11 0160

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | SANO, M. ET AL: "Novel antiallergic catechin derivatives isolated from Oolong tea" JOURNAL AGRIC. FOOD CHEM., vol. 47, no. 5, 1999, pages 1906-1910, XP000824113 * the whole document * --- | 1-7 | |
| A | PATENT ABSTRACTS OF JAPAN vol. 0182, no. 09 (C-1190), 13 April 1994 (1994-04-13) & JP 06 009391 A (MITSUI NORIN KK), 18 January 1994 (1994-01-18) * abstract * --- | 1-7 | TECHNICAL FIELDS SEARCHED (Int.Cl.7) |
| A | PATENT ABSTRACTS OF JAPAN vol. 0153, no. 88 (C-0872), 2 October 1991 (1991-10-02) & JP 03 157330 A (ITOUEN KK), 5 July 1991 (1991-07-05) * abstract * --- | 1-7 | |
| A | DATABASE CHEMABS 'Online! CHEMICAL ABSTRACTS SERVICE, COLUMBUS, OHIO, US; AN: 130:351456, XP002149539 * abstract * & SAIJO, R. ET AL: "HPLC analysis of catechins in various kinds of green teas produced in Japan and abroad" NIPPON SHOKUHIN KAGAKU KOGAKU KAISHI, vol. 46, no. 3, 1999, page 138-147 ----- | 1-7 | |

EPO FORM 1503 03.82 (P04C10)

# ANNEX TO THE EUROPEAN SEARCH REPORT ON EUROPEAN PATENT APPLICATION NO. EP 00 11 0160

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2000

| Patent document cited in search report | Publication date | Patent family member(s) | Publication date |
|---|---|---|---|
| JP 06009391 A | 18-01-1994 | NONE | |
| JP 03157330 A | 05-07-1991 | JP 3023962 B | 21-03-2000 |

EPO FORM P0459

For more details about this annex : see Official Journal of the European Patent Office, No. 12/82